# EUROPEAN PATENT APPLICATION

(11) **EP 1 283 200 A2**
(43) Date of publication of application: **12.02.2003**
(21) Application number: 02380156.6
(22) Date of filing: 10.07.2002
(51) Int. Cl.: C07D 211/22, C12P 41/00, C12P 17/12

(54) **Optically pure paroxetine precursors**

(30) Priority: 13.07.2001 ES 200101648
(71) Applicant: Astur-Pharma, S.A., Llanera 33192, Asturias (ES); Universidad De Oviedo, Llanera 33192, Asturias (ES)
(72) Inventor: Bayod Jasanada, Michael, Silvota Llanera 33192 Austurias (ES); Sanchez Pedrega, Victor M, Silvota Llanera 33192 Austurias (ES); Gotor Santamaria, Vicente, Silvota Llanera 33192 Austurias (ES); Brieva Collado, M. Rosario, Silvota Llanera 33192 Austurias (ES); De Gonzalo Calvo, Gonzalo, Silvota Llanera 33192 Austurias (ES)
(74) Representative: Isern Jara, Nuria

(57) **Abstract**

A biocatalytic process to obtain optically enriched derivatives of trans-4-(4-fluorophenyl)-3-hydroxymethylpiperidines, based on the enzymatic resolution of racemic precursors of formula III (where R³ is preferably phenyl or benzyl) by acylation of the hydroxyl group. Depending on the enzyme and the reaction conditions, either of the two enantiomers may be obtained with high enantiomeric purity. These compounds are important intermediates in the synthesis of the paroxetine anti-depressive agent.

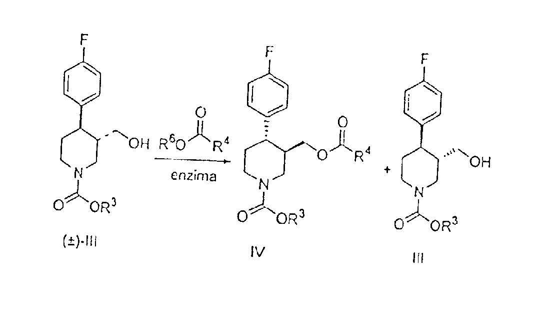

## Description

### Field of the Invention

Paroxetine is an arylpiperidine acting as a selective inhibitor for serotonin (5-HT) re-uptake. Mainly, this compound has anti-depressive and anti-parkinsonian activity. Only the *trans*-(-) isomer, with absolute configuration (3S, 4R) may be used as a drug.

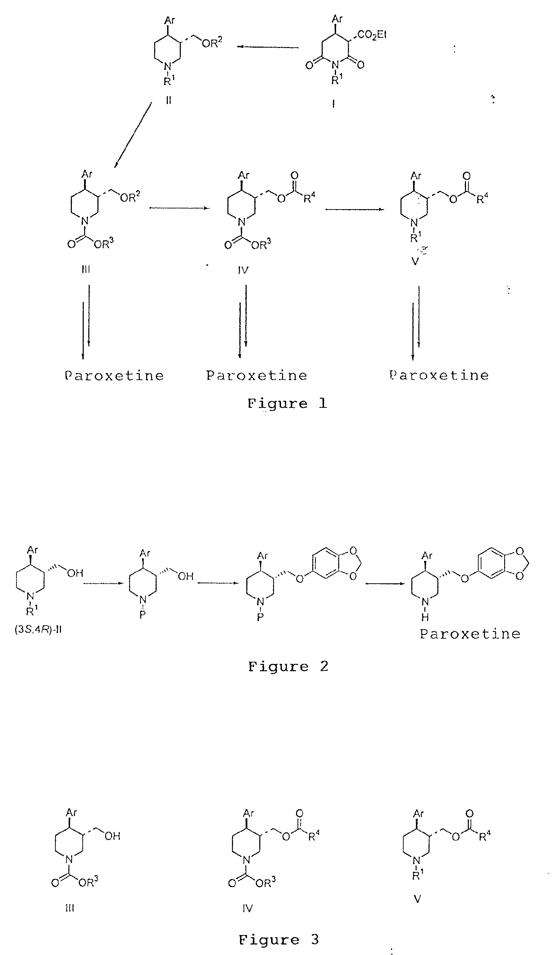

The object of the present invention is a biocatalytic process to obtain optically enriched derivatives of *trans*-4-(4-fluorophenyl)-3-hydroxymethylpiperidines with formulas II, III, IV and V, wherein Ar is a phenyl group, substituted by one or more halogen atoms, R¹ is hydrogen, alkyl or aralkyl, R² is hydrogen, R³ is alkyl, alkenyl, aralkyl or aryl, R⁴ is hydrogen, alkyl, alkenyl, aralkyl or aryl (Figure 1). These are important intermediates in the synthesis of paroxetine.

Another object of the invention are the new compounds obtained, III, IV and V both in the racemic and enatiomerically enriched forms, suitable for the synthesis of paroxetine, as well as their preparation processes. Prior State-of-the-art

### Prior Art

The piperidinecarbinol compounds of formula II, were described for the first time, together with their preparation, in US patents 3,912,743 and 4,007,196 (R¹=hydrogen or methyl and R²=hydrogen). Paroxetine is prepared from said optically pure precursors (3S, 4R)-*trans*-II by means of the process described in US patent 4,007,196. In patent application EP 812 827-A1 and in *Tetrahedron: Asymm*. *1996, 7, 1591,* the preparation of paroxetine from a piperidinecarbinol of formula II is described, with R¹=hydrogen (Figure 2; P=R¹ or other protector group).

In US patents 4,007,196 and 4,902,801, said piperidinecarbinol II compound is firstly obtained as a racemic mixture and then resolved in its enantiomers by conventional methods, such as the crystallisation of its diastereoisomer salts with chiral acids, like (+)-tartaric, (+)-2'-nitrotartranylic or (-)-di-P-toluyltartaric. The drawback of this process is that crystallisation is cumbersome and relatively expensive.

An alternative to said crystallisation of diastereoisomer salts are the biocatalytic enantiomer separation methods.

In US patent 5,258,517 and patent applications WO 93/22284-A1 and WO 98/02556-A3, resolution methods are described for enantiomers of the intermediate I (R¹= Me) by means of enzymatic or microbial hydrolysis. After resolution, the imidoester (3S, 4R)-*trans*-1, enantiomerically enriched, is reduced by conventional methods to piperidinecarbinol (3S, 4R)-*trans*-II.

In patent application WO 98/53824-A1, the preparation of optically enriched (3S, 4R)-*trans*-II (R¹=benzyl) is described, by means of synthesis from an enantiopure precursor, in turn, obtained by means of the enzymatic asymmetrisation of dimethyl 3-(4-fluorophenyl)-glutarate.

On the other hand, derivatives of the piperidinecarbonil carbamate type (general formula III) are described in patent applications WO 97/24323-A1, EP 812 827-A1 and in WO 98/53824-A1. In all these cases, the optically enriched compounds (3S, 4R)-*trans*-III are obtained by means of conventional reactions from an optically pure (3S, 4R)-*trans*-II compound with R² equal to hydrogen. In said patents, paroxetine synthesis is described as from precursors of formula III with R² equal to hydrogen and different R³ substituents.

### General Description of the Invention

For the first time, the present invention describes new compounds of formulas *trans*-IV and *trans*-V, both in the racemic and enantiomerically enriched forms (Figure 3), where:
R¹: hydrogen, alkyl or aralkyl.
R³: alkyl, haloalkyl, alkenyl, aralkyl or aryl.
R⁴: hydrogen, alkyl, haloalkyl, alkenyl, aralkyl or aryl.
Ar: Phenyl group substituted with one or more halogen atoms.

Another object of the present invention is an enzymatic resolution process for piperidinecarbinol derivatives of formula (±)-*trans*-III, in turn permitting to obtain compounds with enantiomerically enriched structures III and IV. Said process consists of the enantioselective acylation -catalysed by an enzyme- of the hydroxymethyl group of the (±)-*trans*-III compound to give a compound of *trans*-IV formula. This process has two variants, permitting the attainment, as required, of either of the two enantiomers of the *trans*-III compound of enantiomierically enriched forms, whether (3S, 4R)-III or (3R, 4S)-III. In the same process, the corresponding *trans*-IV isomer is also obtained with the opposite configuration (3R, 4S)-IV or (3S, 4R)-IV, respectively.

Another object of the present invention is also a preparation process of compounds with formula (3S, 4R)-III, as from compounds of formula (3S, 4R)-IV.

Another object of the present invention is a process to prepare *trans*-V formula compounds from *trans*-IV formula compounds, both in the racemic form and in the enantiomerically enriched form, where R¹ is hydrogen, alkyl or aralkyl.

Another object of the present invention is also a process to prepare optically enriched *trans*-III formula compounds from (3S, 4R)-V formula compounds, consisting of the deacylation of the hydroxymethyl group to give a compound with formula (3S, 4R)-II and the further alcoxycarbonylation of the piperidine nitrogen.

The piperidinecarbinol derivatives of formula III with configuration (3S, 4R) may be used to obtain paroxetine by known methods, already described in the literature.

### Detailed Description of the Invention

The process object of the present invention, consists of making a racemic compound of formula *trans*-III react with an acylating agent in the presence of an enzyme, where R³ and R⁴ represent that mentioned previously and R⁶ may be alkyl, haloalkyl, alkenyl, aralkyl, aryl, iminoalkyl or iminoaryl. By action of the enzyme, one of the enantiomers of the substrate is selectively acylated, obtaining the *trans*-IV ester, while the other enantiomer mainly remains without acylation.

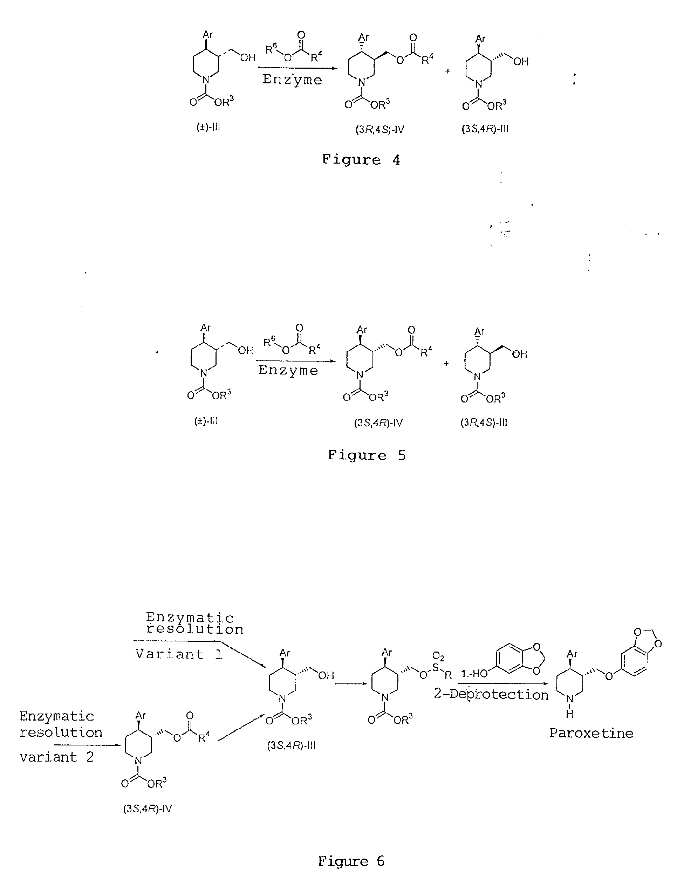

In the first variant of the process (Figure 4), the enzyme preferably catalyses acylation of the enantiomer with configuration (3R, 4S)-III giving a compound (3R, 4S)-IV, while the isomer (3S, 4R)-III mainly remains without acylation.

In a second variant of the process (Figure 5), the enzyme preferably catalyses acylation of the enantiomer with configuration (3S, 4R)-III, giving a compound (3S, 4R)-IV, while the isomer (3R, 4S)-III, mainly remains without acylation.

In either of the two variants of the enzymatic process, when the desired conversion is reached, normally about 50%, the reaction is stopped -for example, by filtering the enzyme- and the resulting compounds are separated. It should be considered that the choice of the enzyme and the specific reaction conditions determine which of the enantiomers is preferably acylated.

When the reaction is sufficiently enantioselective, the reaction conversion should be about 50% to obtain the maximum yield of acylated product and remaining optically enriched substrate. However, when the enantioselectivity is moderate, other conversion values may be preferable to ensure an enantiomeric excess value, sufficiently high for any of the components of the reaction mixture. For example, it is known that as conversion increases under the given reaction conditions, the enantiomeric excess of the remaining substrate gradually increases and the enantiomeric excess of the product gradually decreases. The specific conversion value at which the reaction should be stopped will depend on the enantioselectivity of each specific case and the optical purity requirements of the products. Said value is determined by a known method by an expert in the matter, as described, for example in *J. Amer. Chem. Soc. 1982, 104, 7294*.

The process is carried out by dissolving the substrate in a suitable solvent and adding the enzyme and acylating agent. As a reaction medium, a pure organic solvent or mixture of organic solvents may be used. Suitable solvents may be aliphatic or aromatic hydrocarbon esters, open or cyclic ethers, alcohols, etc. In another variant of the invention, the acylating agent itself may also act as a reaction solvent.

For the acylating agent, activated or inactivated esters are used. This include alkyl, haloalkyl, alkenyl, aryl, aralkyl and oxime esters. The mole ratio of acylating agents and substrate may be equal, less or greater than 1:1.

Enzymes suitable to catalyse this process are the hydrolases, both of animal and microbial origin, in pure or semipurified form, free or immobilised. A special object of the present invention is the use of lipases.

The reaction should be performed between -20 and 100°C, preferably temperatures being between 0°C and 60°C.

It is a known fact, described for example in *Tetrahedrom 2000, 56, 2905*, that certain additives like triethylamine or crown ethers, among others, may be beneficial for the course of the enzymatic reaction.

According to the first variant of the process, once the (3S, 4R)-III isomer has been obtained with high enantiomieric richness, this may be transformed into paroxetine by following already known processes, like the mesylation of the hydroxymethyl group followed by the nucleophillic attack of the 3,4-(methylenedioxy)phenol and final deprotection of nitrogen.

However, in the second variant of the enzymatic process, the enantiomer having the correct configuration to be converted into paroxetine, the (3S, 4R), is acylated by the enzyme to give optically enriched (3S, 4R)-IV. After separation of the compounds (3S, 4R)-IV and (3R, 4S)-III from the reaction mixture, in order to continue with the synthesis it is necessary to deacylate the hydroxymethyl, group of (3S, 4R)-IV, hence transforming it into (3S, 4R)-III. This compound (3S, 4R)-III with high enantiomeric richness, may be converted into paroxetine by following already known processes, like mesylation of the hydroxymethyl group followed by the nucleophillic attack of the 3,4-(methylenedioxy)phenol and final deprotection of the nitrogen. (See figure 6, Ar = *P*-fluorophenyl).

In general, formula III compounds, both in the racemic and enantiomerically enriched forms, may also be obtained from those of structure IV by means of known oxygen deprotection methods (for example, acid or alkaline hydrolysis, aminolysis or alcoholysis), choosing the most suitable method in each case according to R³ and R⁴ structures.

The formula V compounds (R¹=H), both in the racemic and enantiomerically enriched forms, may be obtained from those of structure IV by means of known nitrogen deprotection methods (for example, hydrolysis or hydrogenolysis), choosing the most suitable method in each case according to the R³ and R⁴ structures.

It may occur that in the optically enriched compounds of formula III and IV, the most suitable nitrogen protector group for the enzymatic resolution is not convenient for continuation of the synthesis. In this case, after the enzymatic reaction, the first protector group may be eliminated -giving compounds of formula II and V, respectively- and introducing a new one, aspect which is included in the scope of this invention.

Another object of the present invention are the new acylated compounds in the 3-hydroxymethyl group of the piperidine ring, with formulas IV and V, both in the racemic and enantiomerically enriched forms.

Any new intermediate obtained following the process of this invention constitutes a further aspect of the invention. The processes and products of this invention may be applied to prepare active compounds described in the US patents 3,912,743 and 4,007,196 and preferably to prepare paroxetine.

For a better understanding of the process object of the present invention, the following examples are given, which should be taken as not limiting the scope of the invention.

### Examples

### Example 1

### (±)-trans-N-phenyloxycarbonyl-4-(4-fluorophenyl)-3-hydroxymethylpiperidine (III, R³ = Ph).

1 g of (±)-*trans*-4-(4-fluorophenyl)-3-hydroxymethylpiperidine is dissolved in 15 ml dichloromethane. 15 ml of an aqueous solution of sodium carbonate (2.14 g) are added. At 0°C, 1.25 ml phenyl chloroformiate are added and stirred for one hour. Then, extracted with 15 ml dichloromethane, mixing the organic phases and washing with 5% sodium bicarbonate. The organic phase is concentrated to dryness and is purified by chromatography on silica gel. The product may be crystallised in an ether-hexane mixture. Yield: 68%. White solid, mp.: 129-131°C.
IR (cm⁻¹): 3435, 1705, 1509.
¹H-NMR (CDCL₃)δ (ppm): 1.71-1.94 (m, 4H); 2.50-2.61 (m, 1H); 2.65-3.05 (m,2H); 3.22-3.28 (dd, 1H); 3.41-3.48 (dd, 1H); 4.36-4.57 (m, 2H); 6.99 (dd, 2H); 7.01-7.20 (m, 2H); 7.36 (d, 2H).
¹³C-NMR (CDCl₃) δ (ppm): 33.7 (CH₂); 43.4 (CH); 43.6 (CH); 44.5 (CH₂); 47.2 (CH₂); 62.6 (CH₂); 115.2-115.6 (CH); 121.6 (CH); 125.2 (CH); 128.4-128.5 (CH); 129.2 (CH); 138.8 (C); 151 (C); 153.5 (CO); 159.6-162.9 (C). EM-ESI+: 330 (M+H, 15%); 352 (M + Na, 19); 368 (M + K, 30).

### Example 2

### ±-trans-3-acetyloxymethyl-N-benzyloxycarbonyl-4-(4-fluorophenyl)piperidine. (IV, R³=Bn, R⁴=Me).

1 g (±)-*trans-N*-benzyloxycarbonyl-4-(4-fluorophenyl)-3-hydroxymethylpiperidine is dissolved in 90 ml dichloromethane and 0.95 ml pyridine. At 0°C, 1.1 ml acetic anhydride are added and stirred for 8 hours. Then, it is washed with 1N hydrochloric acid and purified by chromatography on silica gel. Yield: 0.96 g (86%). White solid, mp: 114-115°C.
IR (cm⁻¹): 1735, 1691, 1510.
¹H-NMR (CDCL₃)δ (ppm): 1.70-1.78 (m,2H); 1.99 (s,3H); 2.06 (m, 1H); 2.44-2.51 (m, 1H); 2.53-2.87 (m, 2H); 3.59-3.69 (dd, 1H); 3.81-3.88 (dd, 1H); 4.22-4.49 (m, 2H); 5.18 (s, 2H); 6.96 (dd, 2H); 7.11 (dd, 2H); 7.33-7.41 (m, 5H).
¹³C-NMR (CDCl₃) δ (ppm): 20.7 (CH₃); 34.2 (CH₂); 40.6 (CH); 44.4 (CH₂); 44.5 (CH); 47.2 (CH₂); 64.6 (CH₂); 67.2 (CH₂); 115.4-115.7 (CH); 127.9 (CH); 128.1 (CH); 128.5-128.6 (CH); 128.7 (CH); 136.6 (C); 138.4 (C); 155.1 (CO); 159.9-163.2 (C); 170.1 (CO). EM-IE+: 385 (M+, 1%); 342 (M-Ac, 3); 234 (95); 91 (100).

### Example 3

### (±)-trans-N-benzyloxycarbonyl-3-benzoyloxymethyl-4-(4-fluorophenyl)piperidine. (IV, R³=Bn, R⁴= Ph) .

1 g of (±)-*trans-N*-benzyloxycarbonyl-4-(4-fluorophenyl)-3-hydroxymethylpiperidine is dissolved in 90 ml dichloromethane and 0.95 ml pyridine. At 0°C, 1.35 ml benzoyl chloride is added and stirred for 14 hours. Then, it is washed with 1N HCl and purified by chromatography on silica gel. Yield: 0.91 g (70%), oil.
IR (cm⁻¹) : 1692; 1510. ¹H-NMR (CDCl₃)δ (ppm): 1.64-1.84 (m, 2H); 2.18 (m, 1H); 2.56-2.63 (m, 1H); 2.76-2.92 (m, 2H); 3.86-3.95 (dd, 1H); 4.08-4.15 (dd, 1H); 4.29-4.38 (m, 1H); 4.41-4.55 (m, 1H); 5.22 (s, 2H); 6.96 (dd, 2H); 7.13 (dd, 2H); 7.27-7.47 (m, 6H); 7,57 (d, 2H); 7.96 (d, 2H).
¹³C-NMR (CDCl₃) δ (ppm): 34.1 (CH₂); 41.2 (CH); 44.5 (CH₂); 44.7 (CH); 47.3 (CH₂); 65.0 (CH₂ ); 67.2 (CH₂); 115.5-115.8 (CH); 127.8(CH); 128.0 (CH); 128.3-128.5 (CH); 128.6-128.7 (CH); 128.8 (CH); 129.4 (C); 129.8 (CH); 130.5 (CH); 133.0 (CH); 136.6 (C); 138.4 (C); 155.1 (CO); 160-163.3 (C); 166.1 (CO). EM-IE+: 447 (M+, 3%); 312 (M-Cbz, 10); 234 (97); 91 (100).

### Example 4

### Enzymatic resolution of the intermediate III. (R³= Bn, R⁴= Me).

0.1 g of (±)-*trans-N*-benzyloxycarbonyl-4-(4-fluorophenyl)-3-hydroxymethylpiperidine is dissolved in a suspension of 17 ml toluene and 0.125 g lipase CAL-B. 0.32 ml isopropylene acetate is added and stirred at 30°C. When a conversion close to 50% is reached, the enzyme is filtered and concentrated to dryness. The resulting oil is purified by chromatography on silica gel obtaining (3S, 4R)-*trans-N*-benzyloxycarbonyl-4-(4-fluorophenyl)-3-hydroxymethylpiperidine (99% ee) and (3R, 4S)-*trans*-3-acetyloxymethyl-*N*-benzyloxycarbonyl-4-(4-fluorophenyl) piperidine (79% ee). Conversion: 56%.

### Example 5

### Enzymatic resolution of the intermediate III. (R³= Bn, R⁴= Me).

0.1 g of (±)*-trans-N*-benzyloxycarbonyl-4-(4-fluorophenyl)-3-hydroxymethylpiperidine is dissolved in a suspension of 14.5 ml tert-butylmethylether and 0.6 g lipase of *Mucor miehel*. 0.27 ml vinyl acetate is added and stirred at 30°C. When the desired conversion is reached, the enzyme is filtered and concentrated to dryness. The resulting oil is purified by chromatography on silica gel obtaining (3S, 4R)-*trans-N*-benzyloxycarbonyl-4-(4-fluorophenyl)-3-hydroxymethylpiperidine (13% ee) and (3R, 4S)-*trans*-3-acetyloxymethyl-*N*-benzyloxycarbonyl-4-(4-fluorophenyl)
piperidine (66% ee).
Conversion: 16%.

### Example 6

### Enzymatic resolution of the intermediate III. (R³= Bn, R⁴= Me).

0.1 g (±)-*trans-N*-benzyloxycarbonyl-4-(4-fluorophenyl)-3-hydroxymethylpiperidine is dissolved in a suspension of 17 ml toluene and 0.125 g lipase CAL-A. 0.27 ml vinyl acetate is added and stirred at 15°C. When a conversion close to 50% is reached, the enzyme is filtered and concentrated to dryness. The resulting oil is purified by chromatography on silica gel, obtaining (3R, 4S)*-trans-N*-benzyloxycarbonyl-4-(4-fluorophenyl)-3-hydroxymethylpiperidine (88% ee) and (3S, 4R)-*trans*-3-acetyloxymethyl-*N*-benzyloxycarbonyl-4-(4-fluorophenyl) piperidine (96% ee) [α]ₚ¹⁸= -5.1, c 0.8, MeOH). Conversion: 48%.

### Example 7

### Enzymatic resolution of the intermediate III (R³= Bn, R⁴= Me)

0.1 g of (±)-*trans-N*-benzyloxycarbonyl-4-(4-fluorophenyl)-3-hydroxymethylpiperidine is dissolved in a suspension of 9 ml vinyl acetate and 0.110 g lipase PS-C. Stirred at 30°C. When a suitable conversion is reached, the enzyme is filtered and concentrated to dryness. The resulting oil is purified by chromatography on silica gel, obtaining (3S, 4R)-*trans-N*-benzyloxycarbonyl-4-(4-fluorophenyl)-3-hydroxymethylpiperidine (23% ee) and (3R, 4S)-*trans*-3-acetyloxymethyl-*N*-benzyloxycarbonyl-4-(4-fluorophenyl)
piperidine (82% ee). Conversion: 22%.

### Example 8

### Enzymatic resolution of the intermediate III. (R³= Bn, R⁴= Ph).

0.1 g of (±)-*trans*-*N*-benzyloxycarbonyl-4-(4-fluorophenyl)3-hydroxymethylpiperidine is dissolved in a suspension of 17 ml toluene, 0.4 ml vinyl benzoate and 0.125 g lipase CAL-B. Stirred at 30°C until a suitable conversion is obtained, the enzyme is filtered and concentrated to dryness. The resulting oil is purified by chromatography on silica gel obtaining (3S, 4R)*-trans-N*-benzyloxycarbonyl-4-(4-fluorophenyl)-3-hydroxymethylpiperidine (98% ee) and (3R, 4S)-*trans-N*-benzyloxycarbonyl-3-benzoyloxymethyl-4-(4-fluorophenyl) piperidine (93% ee). [α]_{d}¹⁸= -5.24, c 1.03, MeOH). Conversion: 51%.

### Example 9

### Enzymatic resolution of the intermediate III. (R³= Ph, R⁴= Me).

0.1 g of (±)-*trans-N*-phenyloxycarbonyl-4-(4-fluorophenyl)-3-hydroxymethylpiperidine is dissolved in a suspension of 18 ml toluene, 0.28 vinyl acetate and 0.130 g lipase CAL-A. Stirred at 30°C until a suitable conversion is obtained, the enzyme is filtered and concentrated to dryness. The resulting oil is purified by chromatography on silica gel, obtaining (3R, 4S)-*trans*-*N*-phenyloxycarbonyl-4-(4-fluorophenyl)-3-hydroxymethylpiperidine (97% ee); [α]_{d}¹⁸= +3.89, c 1.36, MeOH) and (3S, 4R)-*trans-N*-phenyloxycarbonyl-3-acetyloxymethyl-4-(4-fluorophenyl) piperidine (92% ee); [α]_{d}¹⁸= -5.03, c 0.74, MeOH). Conversion: 51%.
(3S, 4R)-*trans-N*-phenyloxycarbonyl-3-acetyloxymethyl-4-(4-fluorophenyl) piperidine:
- IR (cm⁻¹): 1714 (broad, 2 signals), 1510.
- ¹H-NMR (CDCl₃)δ (ppm): 1.76-1.90 (m,2H); 2.00 (s,3H); 2.07-2.14 (m, 1H); 2.52-2.61 (m, 1H); 2.63-2.92 (m, 2H); 3.74 (dd, 1H); 3.88 (dd, 1H); 4.42-4.56 (m, 2H); 7.02 (dd, 2H); 7.14-7.36 (m, 5H); 7.47 (t, 2H).
- ¹³C-NMR (CDCl₃) δ (ppm): 20.6 (CH₃); 33.9 (CH₂); 40.7 (CH); 44.3 (CH); 44.6 (CH₂); 47.1 (CH₂); 64.3 (CH₂); 115.4-115.8 (CH); 121.6(CH); 125.2 (CH); 128.4-128.5 (CH); 129.1 (CH); 138.2 (C)'; 151.2 (C); 153.5 (CO); 160.0-163.2 (C); 170.7 (CO).
- EM-ESI+: 372 (M+H, 6%); 394.1 (M+Na, 100); 410 (M+K, 30).

### Example 10

### Enzymatic resolution of the intermediate III. (R³= Bn, R⁴= Me).

0.1 g of (±)-*trans-N*-benzyloxycarbonyl-4-(4-fluorophenyl)-3-hydroxymethylpiperidine is dissolved in a suspension of 17 ml toluene and 0.125 g lipase CAL-A. 0.29 ml ethyl acetate is added. Stirred at 50°C until a suitable conversion is obtained, the enzyme is filtered and concentrated to dryness. The resulting oil is purified by chromatography on silica gel obtaining (3R, 4S)-*trans-N*-benzyloxycarbonyl-4-(4-fluorophenyl)-3-hydroxymethylpiperidine (28% ee) and (3S, 4R)-*trans-N*-benzyloxycarbonyl-3-acetyloxymethyl-4-(4-fluorophenyl) piperidine (95% ee). Conversion: 21%.

### Example 11

### Enzymatic resolution of the intermediate III. (R³= Bn, R⁴= Me).

0.1 g of (±)*-trans-N*-benzyloxycarbonyl-4-(4-fluorophenyl)-3-hydroxymethylpiperidine is dissolved in a suspension of 17 ml toluene and 0.125 g lipase CAL-A. 1.7 g acetonoxime acetate is added and stirred at 30°C. When a conversion of about 50% is obtained, the enzyme is filtered and concentrated to dryness. The resulting oil is purified by chromatography on silica gel obtaining (3R, 4S)-*trans*-*N*-benzyloxycarbonyl-4-(4-fluorophenyl)-3-hydroxymethylpiperidine (92% ee) and (3S, 4R)-*trans*-3-acetyloxymethyl-*N*-benzyloxycarbonyl-4-(4-fluorophenyl) piperidine (83% ee). Conversion: 53%.

### Example 12

### (±)-trans-N-benzyloxycarbonyl-4-(4-fluorophenyl)-3-hydroxymethylpiperidine.

100 mg of (±)-*trans*-3-acetyloxymethyl-*N*-benzyloxycarbonyl-4-(4-fluorophenyl) piperidine are dissolved in 6 ml dry MeOH. 10 ml sodium methoxide solution are slowly added in 0.2 ml methanol at 0°C. After 1 hour stirring at 0°C, it is allowed to heat to room temperature and Dowex 50x4-400 is added until neutral or slightly acid pH. The resin is filtered, washing with MeOH and evaporated to dryness obtaining 78.4 mg of (±)-*trans*-*N*-benzyloxycarbonyl-4-(4-fluorophenyl)-3-hydroxymethylpiperidine. Yield: 88%.

### Example 13

### (3S, 4R)-trans-4-(4-fluorophenyl)-3-hydroxymethylpiperidine.

1g of (3S, 4R)-*trans-N*-benzyloxycarbonyl-4-(4-fluorophenyl)-3-hydroxymethylpiperidine is dissolved in 50 ml ethanol and 300 mg palladium catalyst over carbon in a hydrogen atmosphere is added. On completion of the reaction, it is filtered over Celite and concentrated to dryness. Yield: 87%; [α]_{d}¹⁸= -24.3 (c 0.46, MeOH).

### Example 14

### (3S, 4R)-trans-4-(4-fluorophenyl)-3-acetoxymethylpiperidine.

1g of (3S, 4R)*-trans-N*-benzyloxycarbonyl-4-(4-fluorophenyl)-3-acxetoxymethylpiperidine is dissolved in 50 ml ethanol and 300 mg palladium catalyst over carbon in a hydrogen atmosphere is added. On completion of the reaction, it is filtered over Celite and concentrated to dryness. Yield: 60.1%; [α]_{d}¹⁸= -30.9 (c 1.12, MeOH, 92% ee).
- IR (cm⁻¹): 3347, 1510
- ¹H-NMR (CDCl₃)δ (ppm): 2.10-2.25 (m, 5H); 2.63 (m, 1H); 2.93-3.00 (m, 1H); 3.13-3.32 (m, 2H); 3.66-3.89 (m, 3H); 4.03-4.07(d, 1H, *J* = 11.4 Hz); 7.22-7.28 (t, 2H, *J* = 8.4 Hz); 7.46-7.51 (t, 2H, *J* = 8.4 Hz).
- ¹³C-NMR (CDCl₃) δ (ppm): 20.8 (CH₃); 32.4 (CH₂); 40.2 (CH); 43.5 (CH); 45.8 (CH₂); 48.0 (CH₂); 62.9 (CH₂); 116.9-117.2 (CH); 130.6-130.7 (CH); 139.7 (C); 162.1-163.4 (C); 172.8 (CO).

## Claims

1. A *trans-N*-alcoxycarbonyl-4-aryl-3-acyloxymethylpiperidine of formula IV, with configuration (3S, 4R) or (3R, 4S) or a mixture of both, where:
• R³ is: alkyl, alkenyl, aralkyl or aryl.
• R⁴ is: hydrogen, alkyl, haloalkyl, alkenyl, aralkyl or aryl.
• Ar is: phenyl substituted by one or several halogen atoms.

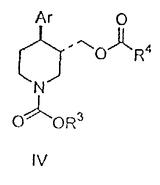

2. A trans-4-aryl-3-acyloxymethylpiperidine of formula V, with configuration (3S, 4R) or (3R, 4S) or a mixture of both, where:
• R¹ is: hydrogen, alkyl, alkenyl, aralkyl or aryl.
• R⁴ is: hydrogen, alkyl, haloalkyl, alkenyl, aralkyl or aryl.
• Ar is: phenyl substituted by one or several halogen atoms.

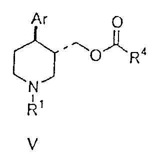

3. A process to prepare a 4-aryl-3-acyloxymethylpiperidine according to claim 1 and a 4-aryl-3-hydroxymethylpiperidine of formula III, both optically active, **characterised by**:
• stereospecifically acylating a mixture of the isomers (+) and (-) of a compound of formula III using an acylating agent of formula R4C(O)OR6 and an enzyme,
• stopping the reaction at a determined conversion, less than 100%, and
• separating the compound of formula (3S, 4R)-IV obtained from the remaining compound (3R, 4S)-III. 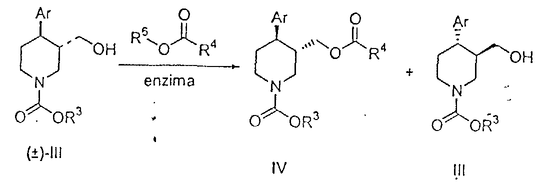
where:
• R³ is: alkyl, alkenyl, aralkyl or aryl.
• R⁴ is: hydrogen, alkyl, haloalkyl, alkenyl, aralkyl or aryl.
• Ar is: phenyl substituted by one or several halogen atoms.
• R⁶ is: alkyl, haloalkyl, aralkyl, alkenyl, aryl, iminoalkyl or iminoaryl

4. A process for the preparation of a 4-aryl-3-acyloxymethylpiperidine according to claim 1 and a 4-aryl-3-hydroxymethylpiperidine of formula III, both optically active, **characterised by**:
• stereospecifically acylating a mixture of the isomers (+) and (-) of a compound of formula III using an acylating agent of formula R4C(O)OR6 and an enzyme.
• stopping the reaction at a determined conversion, less than 100%, and
• separating the compound of formula (3R, 4S)-IV obtained from the remaining (3S, 4R)-III compound. 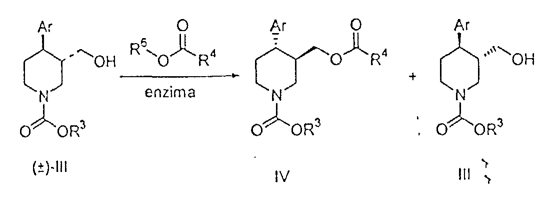
where:
• R³ is: alkyl, alkenyl, aralkyl or aryl.
• R⁴ is: hydrogen, alkyl, haloalkyl, alkenyl, aralkyl or aryl.
• Ar is: phenyl substituted by one or several halogen atoms.
• R⁶ is: alkyl, haloalkyl, aralkyl, alkenyl, aryl, iminoalkyl or iminoaryl

5. A process according to claims 3 and 4, **characterised in that** the final enantiomeric excess of compound IV is greater than 60%.

6. A process according to claims 3 and 4, **characterised in that** the final enantiomeric excess of compound IV is greater than 95%.

7. A process according to claims 3 and 4, **characterised in that** the final enantiomeric excess of compound III is greater than 60%.

8. A process according to claims 3 and 4, **characterised in that** the final enantiomeric excess of compound III is greater than 95%.

9. A process according to claims 3 and 4, **characterised in that** the variable part of the formula R³ is methyl, allyl, benzyl, tert-butyl or phenyl.

10. A process according to claims 3 and 4, **characterised in that** the variable part of the formula R⁴ is hydrogen, methyl or phenyl.

11. A process according to claims 3 and 4, **characterised in that** the variable part of the formula R⁶ is vinyl, isopropenyl or ethyl.

12. A process according to claims 3 and 4, **characterised in that** the R⁴ C(O)OR6 compound is an oxime ester.

13. A process according to claims 3 and 4, **characterised in that** the variable part of the formula Ar is 4-fluorophenyl.

14. A process according to claims 3 and 4, **characterised in that** the enzyme is a hydrolase.

15. A process according to claims 3 and 4, **characterised in that** the enzyme is a lipase.

16. A process according to claims 3 and 4, **characterised in that** the lipase comes from a micro-organism of the genres *Candida, Rhizomucor, Pseudomonas or Aspergillus.*

17. A process according to claims 3 and 4, **characterised in that** the lipase is CAL-A (lipase A of *Candida antarctica*), CAL-B (lipase B of *Candida antarctica*), PS-C (lipase or *Pseudomonas cepacia*) or MML (lipase of *Rhizomucor miehei*).

18. A process according to claims 3 and 4, **characterised in that** the enzyme is partially or totally purified.

19. A process according to claims 3 and 4, **characterised in that** the enzyme is immobilised.

20. A process according to claims 3 and 4, **characterised in that** the reaction is performed in an organic solvent or in a mixture of organic solvents.

21. A process according to claims 3 and 4, **characterised in that** the reaction is performed in any solvent from toluene, ethyl acetate, vinyl acetate or tert-butylmethylether.

22. A process to obtain a compound of formula (3S, 4R)-III, **characterised by** the deacylation (by hydrolysis, alcoholysis or ester aminolysis) of a compound of formula (3S, 4R)-IV obtained according to claim 3.

23. A process for the preparation of paroxetine, **characterised in that** it is obtained from a (3S, 4R)-III compound, in turn obtained according to either of the claims 4 or 22.

24. A process for the preparation of paroxetine, **characterised in that** it is obtained from a (3S, 4R)-IV compound in turn obtained according to claim 3.

25. A process for the preparation of a piperidinecarbinol of formula II, both racemic and enantiomerically enriched, **characterised by** deprotection of the piperidine ring nitrogen of a compound of formula III, obtained according to claims 4 or 22.

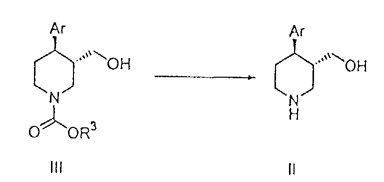

26. A process for the preparation of an acyloxymethylpiperidine of formula V according to claim 2, **characterised by** the deprotection of the piperidine ring nitrogen of a compound of formula IV, obtained according to claim 3.
